# EUROPÄISCHE PATENTSCHRIFT

(11) **EP 0 953 294 B1**
(45) Veröffentlichungstag und Bekanntmachung des Hinweises auf die Patenterteilung: **21.09.2005**
(21) Anmeldenummer: 99107803.1
(22) Anmeldetag: 20.04.1999
(51) Int. Cl.: A23L 1/226, C07D 307/83, A61K 7/16

(54) **Verwendung von 3,6-Dimethyl-2-(3H)-benzofuranon als Aromastoff; Nahrungs-, Genuss- und Mundpflegemittel enthaltend 3,6-Dimethyl-2-(3H)-benzofuranon**
Use of 3,6-Dimethyl-2-(3H)-benzofuranone as a flavouring agent, food and oral care compositions comprising 3,6-dimethyl-2-(3H)-benzofuranon
Utilisation de 3,6-Dimethyl-2-(3H)-benzofuranone comme agent aromatisant, compositions alimentaires et orales comprenant le 3,6-dimethyl-2-(3H)-benzofuranone

(30) Priorität: 27.04.1998 DE 19818731; 06.03.1999 DE 19909980
(43) Veröffentlichungstag der Anmeldung: 03.11.1999
(73) Patentinhaber: Symrise GmbH & Co. KG, 37603 Holzminden (DE)
(72) Erfinder: Lambrecht, Stefan Dr., 37603 Holzminden (DE); Surburg, Horst Dr., 37603 Holzminden (DE); Güntert, Matthias Dr., 37603 Holzminden (DE); Koppe, Volkmar, 37603 Holzminden (DE)
(74) Vertreter: Eisenführ, Speiser & Partner

(56) Entgegenhaltungen:
- WO-A-95/30667
- DE-A- 3 017 068

## Beschreibung

Die Erfindung betrifft die Verwendung von 3,6-Dimethyl-2(3H)-benzofuranon zum Herstellen von Aromen, insbesondere Vermitteln einer lactonigen, cumarinigen und fruchtigen Note, entsprechende Mintkompositionen sowie entsprechende Nahrungs-, Genuß- und Mundpflegemittel.

3,6-Dimethyl-2(3H)-benzofuranon ist als Riechstoff für Parfümkompositionen bekannt (WO 95/30667).

Außerdem sind Verfahren zur Herstellung von 3,6-Dimethyl-2(3H)-benzofuranon bekannt, bei denen mit chlorhaltigen Reagenzien und Lösungsmittel gearbeitet wird, die in ihrer Anwendung problematisch sind und eine technische Herstellung erschweren.

Es wurden Aromen gefunden, die 3,6-Dimethyl-2(3H)-benzofuranon der Formel 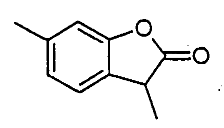 enthalten.

Überraschenderweise stellte sich heraus, daß diese Verbindung sich durch neue und bislang unbekannte Geschmacksnoten von bekannten Aromastoffen vorteilhaft unterscheiden.

Das erfindungsgemäß 3,6-Dimethyl-2(3H)-benzofuranon enthaltende Aroma liegt bevorzugt in flüssiger, sprühgetrockneter oder verkapselter Form vor.

Die Substanz weist ein besonders intensives cumariniges, heuartiges und kokosartiges Aroma mit einer lang andauernden bemerkenswerten Geschmacksfülle auf. Diese Eigenschaft gewinnt dadurch besondere Bedeutung, daß Cumarin selbst als Aromastoff für die Herstellung von Lebensmittelaromen nicht verwendet werden darf (EG-Richtlinie v. 22.6.1988). Außerdem gehört Cumarin zu den sogenannten "active principles", d.h. zu den Verbindungen, die in Lebensmitteln höchstmengenbegrenzt sind. Verbindungen mit einem cumarin-artigen Geschmacksprofil sind daher begehrte Aromastoffe, mit denen sich Aromen auch ohne die Verwendung von Cumarin die cumarin-typischen süßen, lactonigen und heuartigen Noten verleihen lassen.

Erfindungsgemäß eignet sich die Verbindung 3,6-Dimethyl-2(3H)-benzofuranon wegen ihres herausragenden organoleptischen Charakters vorzüglich als Aromastoff für den Einsatz in Aromakompositionen, wobei sie den betreffenden Aromen eine sehr intensive naturhafte cumarinige und heuartige Note verleiht. Bemerkenswert ist außerdem die dabei durch 3,6-Dimethyl-2(3H)-benzofuranon hervorgerufene Süße und Geschmacksfülle. Im Vergleich zu den schon als Aromastoffen verwendeten Lactonen 5,6,7,7a-Tetrahydro-3,6-dimethyl-2(4H)-benzofuranon und 5,6-Dihydro-3,6-dimethyl-2(4H)-benzofuranon ist die Verbindung 3,6-Dimethyl-2(3H)-benzofuranon von einer höheren geschmacklichen Intensität, d.h. bei vergleichbarer Dosierung tritt das unter Verwendung von 3,6-Dimethyl-2(3H)-benzofuranon hergestellte Aroma schneller, deutlicher und kräftiger hervor ("Impactwirkung").

Der geschmackliche Eindruck einer Aromakomposition wird also durch die Zugabe von 3,6-Dimethyl-2(3H)-benzofuranon erheblich verstärkt und aufgewertet, was letztlich zu einer vergleichsweise höheren Akzeptanz der Fertigprodukte führt, die solche Aromen enthalten.

In Aroma-Kompositionen beträgt die eingesetzte Menge der Verbindung 3,6-Dimethyl-2(3H)-benzofuranon erfindungsgemäß vorzugsweise 0,005 bis 10 Gew.-%, insbesondere 0,01 bis 5 Gew.-%, bezogen auf die gesamte Komposition. Derartige Aroma-Kompositionen können im gesamten Nahrungs- und Genußmittelbereich sowie in Produkten für die Mundpflege verwendet werden. Insbesondere sind sie geeignet für die Aromatisierung von Fettmassen, Backwaren, Joghurt, Speiseeis, Süßwaren, Kaugummi, alkoholische und nicht alkoholische Getränke, Tabak, Zahnpasta und Mundwässern. Die Dosierung derartiger Aroma-Kompositionen liegt vorzugsweise bei 0,0005 bis 2 Gew.-%, insbesondere bei 0,01 bis 1 Gew.-%, bezogen auf das fertige Nahrungs- oder Genußmittel.

In zunehmendem Maße sind in der Aromen- und Lebensmittelindustrie aber auch Stoffe wichtig, die neben der Eigenschaft, einen bestimmten Geruch und Geschmack zu verleihen, zusätzliche Eigenschaften aufweisen. Dies können zum Beispiel bestimmte Reize sein, die über den Trigeminus-Nerv geleitet und so wahrgenommen werden. Es können aber auch Effekte sein, durch die Geruchs- und Geschmackesempfindungen gehemmt oder verstärkt werden.

Ein Schwerpunkt der erfindungsgemäßen Verwendung der Verbindung 3,6-Dimethyl-2(3H)-benzofuranon liegt auf ihrem Einsatz in Aromakompositionen für Süßwaren, Kaugummi, und Produkten für die Mundpflege (Zahnpasta, Mundwässer). Insbesondere werden sie in Mintkompositionen verwendet, die üblicherweise aus Pfefferminzölen (*Mentha piperita*), Kornmintölen (*Mentha arvensis*) und/oder Krauseminzölen (*Mentha spicata* und *Mentha cardiaca*) und deren Fraktionen unter Zusatz von synthetischen und natürlichen Aromastoffen und weiteren natürlichen Extrakten oder etherischen Ölen bestehen. Diese Mintaromen verleihen den entsprechend aromatisierten Lebensmitteln und Mundpflegemitteln einen minzigen Geruch und Geschmack und sorgen für Frische im Mundbereich und Atem. Ein wichtiger Teil des Geschmacksprofils von Pfefferminzölen (*Mentha piperita*) sind die süßen, heuartigen Noten (von manchen Testpersonen auch als Tabaknoten bezeichnet), die insbesondere bei der Aromatisierung von Süßwaren und Kaugummi erwünscht sind. Nachstehend ist das Geschmacksprofil eines reinen Pfefferminzöls in Kaugummi aufgeführt, das diese Aussage unterstreicht.

Geschmacksprofil eines Pfefferminzöles (*Mentha piperita*) in Kaugummi (zuckerfrei, Dosierung 1,2%)

| Geschmacksprofil | Intensität (1-10) |
|---|---|
| Impact | 6 |
| Fülle | 8 |
| Frische | 6 |
| Kühle | 6 |
| Eukalyptus | 5 |
| Menthol | 5 |
| Menthon | 4 |
| Grün | 2 |
| Würzig | 6 |
| Tee | 8 |
| Heuig | 7 |
| Kamille | 5 |
| Blütig | 4 |
| Cumarin | 5 |
| Süß | 7 |
| Weich | 8 |
| Erdig | 1 |
| Pilzig | 1 |

Es wurde nun überraschend festgestellt, daß die Verbindung 3,6-Dimethyl-2(3H)-benzofuranon ausgesprochen synergistische geschmackliche Effekte hervorrufen kann. Während sie in ihrer Eigenschaft als Aromastoff die oben beschriebenen geschmacklichen Eigenschaften "cumarinig, kokosartig, heuartig" aufweist, ist sie in Mintkompositionen auch in der Lage, die "süßen" und "krautigen" Noten signifikant zu verstärken und "abrundend" zu wirken

Diese Erkenntnisse wurden in systematischen sensorischen Versuchen mit synthetischen Mintaromen erarbeitet, in denen der erfindundungsgemäße Aromastoff 3,6-Dimethyl-2(3H)-benzofuranon einmal zugesetzt und einmal aus der Komposition herausgelassen wurde. In zuckerhaltigen und nicht-zuckerhaltigen Kaugummibasen wurden die Mintaromen in praxisüblichen Konzentrationen (1-1,5%) eingearbeitet und nach etwa 4 Wochen Lagerzeit verkostet. Der Vergleich der Mintaromen mit und ohne 3,6-Dimethyl-2(3H)-benzofuranon führte zu dem Ergebnis, daß die Verbindung synergistisch wirkt und insbesondere "die Süße verstärkt, die Krautigkeit abrundet, das Menthol abdeckt und generell abrundend wirkt". Die sensorischen Kauversuche wurden bis zu 30 Minuten durchgeführt.

Die Versuche mit Mundpflegemitteln wurden insbesondere an Zahnpasten durchgeführt. Hier wurden sowohl Calciumcarbonat- wie auch Silicatbasen verwendet. Die Basen waren mit 0,2 % Saccharin gesüßt. Auch hier wurden synthetische Mintaromen eingesetzt, in denen der Aromastoff 3,6-Dimethyl-2(3H)-benzofuranon einmal zugesetzt und einmal aus der Komposition herausgelassen wurde. Der Vergleich der Mintaromen mit und ohne 3,6-Dimethyl-2(3H)-benzofuranon führte zu dem Ergebnis, daß die Verbindung synergistisch wirkt und insbesondere "den Impact verstärkt, die Süße verstärkt, die Schärfe des Menthols abdeckt und abrundend wirkt".

Zusammenfassend kann man also sagen, daß die Verbindung 3,6-Dimethyl-2(3H)-benzofuranon in Mintkompositionen in Kaugummi die folgenden synergistischen geschmacklichen Eigenschaften besitzen:
- Verstärkung der Süße
- Verstärkung der Krautigkeit
- Verstärkung des Impacts
- Abrundung der gesamten Komposition.

In Mintkompositionen in Zahnpasta besitzt die Verbindung 3,6-Dimethyl-2(3H)-benzofuranon die folgenden synergistischen geschmacklichen Eigenschaften:
- Verstärkung des Impacts
- Verstärkung der Süße
- Abrundung der Menthol-Schärfe
- Abrundung der gesamten Komposition

Die zur Anwendung kommenden Mintaromen, die erfindungsgemäß den Aromastoff 3,6-Dimethyl-2(3H)-benzofuranon enthalten, können sowohl in flüssiger wie auch in trockener Form eingesetzt werden. Während sie in flüssiger Form in einem praxisüblichen Lösungsmittel wie Ethanol, Propylenglycol oder Triacetin eingesetzt werden, gewinnt man die trockenen Aromen durch Sprühtrocknung oder durch Verkapselung nach einem der in der Aromenindustrie üblichen Verfahren. Dies sind insbesondere die Extrusion und die Sprühgranulation.

Die Herstellung aromatischer Verbindungen aus gesättigten und teilgesättigten Vorstufen durch katalytische Dehydrierung ist an sich eine bekannte Verfahrensweise in der organischen Chemie (Methoden d. Org. Chemie (Houben-Weyl), 4. Aufl. 1981, Bd. 5/2b). Die Verwendung von α,β-ungesättigte Carbonylverbindungen als Wasserstoffakzeptor ist ebenfalls beschrieben (M.L.A. von Holleben, M. Zucolotto, C.A. Zini, E.R. Oliveira, Tetrahedron, 1994, 50, 973-978). Zur Herstellung von 2(3H)-Benzofuranonen aus entsprechenden partiell hydrierten Vorstufen ist diese Methode aber bisher noch nicht verwendet worden.

Ein solches Verfahren bietet jedoch im Falle von 3,6-Dimethyl-2(3H)-benzofuranon den Vorteil, daß mit 5,6-Dihydro-3,6-dimethyl-2(4H)-benzofuranon ein bereits in technischem Maßstab verfügbares Ausgangmaterial vorhanden ist(DE 3.017.068).

Wie es sich zeigte, gelingt die Herstellung von 3,6-Dimethyl-2(3H)-benzofuranon, wenn man die Dehydrierung in flüssiger Phase durchführt, wobei als Dehydrierungskatalysator ein Metall der 8. Nebengruppe dient und als Wasserstoffakzeptoren α,β-ungesättigte Carbonylverbindungen eingesetzt werden.

Als Dehydrierungskatalysator wird vorzugsweise Palladium verwendet, das auf einem Träger fixiert ist. Der Träger ist vorzugsweise Kohle.

Als Wasserstoffakzeptoren dienen Verbindungen wie Fumarsäureester, Maleinsäureester, Mesityloxid, Benzalaceton, Isophoron, Verbenon, Crotonsäureester usw. Besonders bevorzugt ist die Verwendung von Dehydrierungsmitteln, deren Siedetemperatur so liegt, daß das Produkt entweder deutlich später (z.B. Mesityloxid) oder deutlich früher (z.B. Maleinsäuredibutylester) siedet. Auf diese Weise wird eine einfache Desillation ermöglicht.

Die α,β-ungesättigten Carbonylverbindungen werden im Überschuß eingesetzt und dienen daher gleichzeitig als Lösungsmittel. Auf die Verwendung eines weiteren Lösungsmittels kann daher verzichtet werden.

Die Reaktion läuft bei Temperaturen zwischen 70 und 250°C; bei Mesityloxid wird vorzugsweise unter Rückflußbedingungen bei ca. 130°C gearbeitet. Bei der Verwendung von Maleinsäuredibutylester wird vorzugsweise bei 170°C gearbeitet. Das Verfahren kann unter Normaldruck und unter Über- oder Unterdruck durchgeführt werden.

Die Katalysatormenge kann 0,001 bis 30 % betragen, vorzugsweise 0,2 bis 15 %.

### Beispiele

### Herstellverfahren 1: Herstellung von 3,6-Dimethyl-2(3H)-benzofuranon mit Mesityloxid als Wasserstoffakzeptor

Eine Mischung aus 100 g 5,6-Dihydro-3,6-dimethyl-2(4H)-benzofuranon, 500 ml Mesityloxid und 10 g Palladium auf Kohle, 5 Gew.-%, werden unter Rückfluß zwei Stunden lang erhitzt. Anschließend wurde das Verhältnis von ca. 3:1 von 3,6-Dimethyl-2(3H)-benzofuranon zu 5,6,7,7a-Tetrahydro-3,6-dimethyl-2(4H)-benzofuranon durch Gaschromatographie bestimmt.

Das Reaktionsgemisch hatte folgende Zusammensetzung:

| | |
|---|---|
| Mesityloxid: | 69,7 % |
| 3,6-Dimethyl-2(3H)-benzofuranon | 14,7% |
| 5,6,7,7a-Tetrahydro-3,6-dimethyl-2(4H)-benzofuranon: | 4,6 % |

Zur Reinigung des Produktes wurde das Mesityloxid über eine 20cm-Füllkörperkolonne abdestillert und der Rückstand fraktioniert. Bei einem Sdp. von 110° bei 3 mbar wurden ca. 80 g einer aus 3,6-Dimethyl-2(3H)-benzofuranon und 5,6,7,7a-Tetrahydro-3,6-dimethyl-2(4H)-benzofuranon bestehenden Fraktion aufgefangen, aus der durch Feindestillation über eine Spaltrohrkolonne® reines 3,6-Dimethyl-2(3H)-benzofuranon erhalten wurde..

Spektroskopische Daten (FT/IR):

| | |
|---|---|
| 2979 cm⁻¹ | w |
| 1801 cm⁻¹ | s |
| 1632 cm⁻¹ | m |
| 1501 cm⁻¹ | m |
| 1454 cm⁻¹ | m |
| 1423 cm⁻¹ | m |
| 1096 cm⁻¹ | m |
| 1030 cm⁻¹ | m |
| 994 cm⁻¹ | m |
| 943 cm⁻¹ | m |

### Herstellverfahren 2: Herstellung von 3,6-Dimethyl-2(3H)-benzofuranon durch Dehydrierung mit Maleinsäuredibutylester als Wasserstoffakzeptor

Eine Mischung aus 1000 g 5,6-Dihydro-3,6-dimethyl-2(4H)-benzofuranon, 2000 ml Maleinsäuredibutylester und 50 g Palladium auf Kohle, 5 Gew.-%, wurden unter Eigendruck in einem Autoklaven ca. 3 Stunden auf 170°C erhitzt. Nach der Filtration wurde zur Remigung des Produktes mit einer 80cm-Füllkörperkolonne fraktioniert. Bei einem Sdp. von ca. 90°C bei 1 mbar wurden ca. 735 g reines 3,6-Dimethyl-2(3H)-benzofuranon erhalten wurde.

### Verwendungs Beispiel 1: Herstellung eines Kokos-Aromas

Es wurden vermischt (alle Angaben in g):

| | |
|---|---|
| Acetylmethylcarbinol | 5,0 |
| γ-Nonalacton | 50,0 |
| Benzaldehyd | 0,5 |
| Caprinsäure | 5,0 |
| Vanillin | 5,0 |
| 1,2-Popropylenglycol | 934,5 |
| Summe | 1000,0 |

Ersetzte man 0,5 g des Propylenglycols durch 0,5 g 3,6-Dimethyl-2(3H)-benzofuranon, so wurde das Aroma deutlich typischer in Richtung Kokos und gewann merklich an Duft, Süße, Fülle und Impact.

### Verwendungs Beispiel 2: Herstellung eines Pfefferminz-Aromas

Es wurden vermischt (alle Angaben in g):

| | |
|---|---|
| Eucalyptol | 50 |
| I-Limonen | 20 |
| Menthofuran | 20 |
| I-Menthol | 450 |
| Menthon/Isomenthon | 250 |
| I-Menthylacetat | 40 |
| Alkohol, 96 % vol | 170 |
| Summe | 1000 |

Ersetzt man 1 g des Alkohols durch 1 g 3,6-Dimethyl-2(3H)-benzofuranon, so wurde das Aroma deutlich süßer, lactoniger, cumariniger und gewann an Duft, Fülle und Impact. Außerdem konnte eine Abrundung des Aromas festgestellt werden.

## Patentansprüche

1. Verwendung von 3,6-dimethyl-2(3H)-benzofuranon zum Herstellen von Aromen.

2. Verwendung von 3,6-dimethyl-2(3H)-benzofuranon zum Vermitteln eines cumarinigen, heuartigen und/oder kokosartigen Aromas.

3. Verwendung von 3,6-dimethyl-2(3H)-benzofuranon in einer Aromakomposition zum Verstärken der Süße, der Geschmacksfülle und/oder zum schnelleren, deutlicheren und kräftigeren Hervortretenlassen des Aromas der Aromakomposition.

4. Verwendung von 3,6-dimethyl-2(3H)-benzofuranon in einer Mintkomposition zum Verstärken süßer und/oder krautiger Noten.

5. Mintkomposition, enthaltend 3,6-dimethyl-2(3H)-benzofuranon in einer zum Verstärken einer süßen und/oder krautigen Note und/oder zum Abrunden ausreichenden Konzentration.

6. Nahrungs-, Genuss- und/oder Mundpflegemittel, enthaltend eine Aromakomposition umfassend 3,6-dimethyl-2(3H)-benzofuranon, wobei der Gehalt an Aromakomposition 0,0005 bis 2 Gew.-% bezogen auf das fertige Nahrungs-, Genuss- bzw. Mundpflegemittel beträgt.

7. Mundpflegemittel, enthaltend eine Mintkomposition und 3,6-dimethyl-2(3H)-benzofuranon in einer ausreichenden Konzentration zum Verstärken einer süßen und/oder krautigen Note und/oder in einer zum Abrunden ausreichenden Konzentration.

## Claims

1. Use of 3,6-dimethyl-2(3H)-benzofuranone for the production of flavourings.

2. Use of 3,6-dimethyl-2(3H)-benzofuranone to provide a coumarin-like, hay-like and/or coconut-like flavour.

3. Use of 3,6-dimethyl-2(3H)-benzofuranone in a flavouring composition to reinforce the sweetness, the fullness of flavour and/or to allow the flavour of the flavouring composition to come to the fore more rapidly, more clearly and more strongly.

4. Use of 3,6-dimethyl-2(3H)-benzofuranone in a mint composition to reinforce sweet and/or herbal notes.

5. Mint composition containing 3,6-dimethyl-2(3H)-benzofuranone in a concentration sufficient to reinforce a sweet and/or herbal note and/or to provide a rounding-off effect.

6. Foodstuffs, products consumed for pleasure and/or oral care products containing a flavouring composition comprising 3,6-dimethyl-2(3H)-benzofuranone, wherein the content of flavouring composition is 0.0005 to 2 wt.%, based on the finished foodstuff, product consumed for pleasure or oral care product.

7. Oral care product containing a mint composition and 3,6-dimethyl-2(3H)-benzofuranone in a concentration sufficient to reinforce a sweet and/or herbal note and/or in a concentration sufficient to provide a rounding-off effect.

## Revendications

1. Utilisation de la 3,6-diméthyl-2(3H)-benzofuranone pour la production d'aromes.

2. Utilisation de la 3,6-diméthyl-2(3H)-benzofuranone pour conférer un arome coumarinigène, de type foin ou de type coco.

3. Utilisation de la 3,6-diméthyl-2(3H)-benzofuranone dans une composition aromatique pour renforcer la douceur, la plénitude du goût et/ou pour la manifestation plus rapide, plus nette et plus puissante de l'arome de la composition aromatique.

4. Utilisation de la 3,6-diméthyl-2(3H)-benzofuranone dans une composition de menthe pour renforcer des notes douces et/ou herbacées.

5. Composition de menthe contenant de la 3,6-diméthyl-2(3H)-benzofuranone en une concentration suffisante pour renforcer une note douce et/ou herbacée et/ou pour arrondir.

6. Produit alimentaire, de consommation et/ou d'hygiène de la bouche, contenant une composition aromatique comprenant de la 3,6-diméthyl-2(3H)-benzofuranone, où la teneur en composition aromatique est 0,0005 à 2 % en masse par rapport au produit alimentaire, de consommation ou d'hygiène de la bouche prêt.

7. Produit d'hygiène de la bouche contenant une composition de menthe et de la 3,6-diméthyl-2(3H)-benzofuranone en une concentration suffisante pour renforcer une note douce et/ou herbacée et/ou en une concentration suffisante pour arrondir.
